# EUROPEAN PATENT APPLICATION

(11) **EP 2 387 938 A1**
(43) Date of publication of application: **23.11.2011**
(21) Application number: 10275054.4
(22) Date of filing: 20.05.2010
(51) Int. Cl.: A61B 3/11, A61B 3/113, A61B 5/18

(54) **Eye monitor for monitoring the size of a pupil**

(71) Applicant: BAE Systems PLC, London SW1Y 5AD (GB)
(72) Inventor: The designation of the inventor has not yet been filed
(74) Representative: BAE SYSTEMS plc Group IP Department

(57) **Abstract**

An eye monitor is disclosed for monitoring the size of a pupil of a monitored subject. The eye monitor comprises tracking means for tracking movement of the user's pupil and monitoring means for monitoring the size of the user's pupil. The eye monitor provides for a continuous and real time analysis of the subject's eye and in particular the pupil of the eye, to determine whether the subject has been exposed to harmful chemicals and without requiring the subject to actively participate in the monitoring.

## Description

The present invention relates to an eye monitor or method of monitoring pupillary response, in particularly, but not exclusively, to an eye monitor for monitoring the size of a pupil of an eye of a monitored subject.

US 6,637,885 discloses a method for the self-detection of exposure to chemicals such as organophosphates and harmful gas, by monitoring pupillary response. The method disclosed requires a user to place an eyeglass cup of a monitoring device over the eye to be tested, blocking the light from entering the other eye and to subsequently observe whether the pupil of the eye under test dilates upon switching the device on.

The device operates on the basis that the pupil of the eye being blocked will tend to dilate, due to reduced levels of light entering the blocked eye. The pupil of the eye under test should respond consensually, i.e. the pupil of the eye under test should also dilate. If the pupil of the eye under test does not dilate then it is possible that miosis (pupil contraction) has occurred, which is indicative of exposure to chemicals.

However, a problem with the device disclosed in US 6,637,885 is that a user must perform frequent checks to determine whether they have been exposed to dangerous gases, and as such, the user must stop performing their current task to carry out the check. This can be a particular problem, particularly for aircraft pilots. In addition, since a pilot may travel large distances in a relatively short time, then it would be necessary to perform the checks more frequently to ascertain whether they have been exposed to dangerous chemicals.

In addition, the device of US 6,637,885 requires a user to cover both eyes for a period of time. This is undesirable when flying an aircraft or during military manoeuvres, when it is necessary to remain alert and vigilant, for example.

In accordance with a first aspect of the present invention, there is provided an eye monitor for monitoring the size of a pupil of an eye of a monitored subject, the monitor including tracking means for tracking movement of the pupil and means for determining the size of the pupil.

In this manner, the detection of the exposure of a subject to harmful substances or chemicals can be undertaken without active input from the subject. The ability of the eye monitor to track the pupil of an eye of a subject provides for a continuous, real time analysis of the pupil to determine whether the subject has been exposed to harmful toxins, for example, and thus can enable a warning to be issued to the subject to take the appropriate action when exposure has occurred.

The tracking means may include a transmitter for transmitting a probe signal substantially upon the eye of the subject and a receiver for receiving a signal reflected off the eye of the subject. The transmitter may include an infrared transmitter for transmitting a probe signal having a wavelength characteristic of infrared wavelengths. The receiver may include an infrared sensor for receiving the probe signal reflected from the eye of the subject. The infrared sensor may be a camera or suitably arranged charge coupled device.

The tracking means may further include signal steering means for steering the transmitted signal upon the eye of the subject. The signal steering means may be further arranged to scan the probe signal across the eye of the subject.

The receiver may be arranged to monitor the variation in intensity of the reflected signal. It is found that the pupil and iris which surrounds the pupil reflect the signal by different amounts. Accordingly, once the centre-point of the pupil is identified, the pupil diameter can be measured by determining the range either side of the centre point which provides substantially the same reflected signal intensity.

The signal steering means may be arranged to direct the probe signal onto the eye of the subject in accordance with the reflected signal intensity.

The means for determining the size of the pupil may include a processor which is arranged to process the monitored variation in intensity of the reflected signal, as determined by the receiver.

The eye monitor may further include control means for controlling the operation of the transmitter and the receiver. The control means and/or the processor may be communicatively coupled with the transmitter and the receiver. The control means and the processor may be disposed upon the eye monitor or upon a pilot's helmet, for example.

According to another aspect of the invention, a head or helmet mountable eye monitor includes an eye monitor mounted upon a frame. For example, a frame for a pair of spectacles. The frame may be arranged to support an optical element in front of one or both of the eyes of the subject. The optical element may include a lens, visor or optical combiner.

The signal steering means may be arranged to steer the probe signal upon the optical element, such that the probe signal will reflect off the optical element, substantially onto one or both of the eyes of the subject. Similarly, the signal reflected from the eye of the subject is arranged to reflect off the optical element before being received at the receiver.

According to another aspect of the invention, a vehicle mountable eye monitor includes an eye monitor mounted to the structure of a vehicle. For example, the eye monitor may be mounted within an aircraft cockpit, a land vehicle such as a tank, armoured vehicle or other transport vehicle such as a passenger bus, train or aircraft.

In accordance with another aspect of the present invention, there is provided a method of monitoring pupillary response, the method including:
- tracking pupil movement to determine pupil position;
- transmitting a probe signal substantially upon the pupil in response to the tracked position; and,
- receiving a signal reflected off the pupil.

The method may further include processing the received signal to determine the pupil size. The method may include comparing the measured pupil size with a calibrated acceptable pupil range. The calibrated pupil range may be that measured under controlled light levels or an average pupil size from a population group to which the eye of the subject under test belongs.

An embodiment of the present invention will now be described by way of example only and with reference to the accompanying drawings, in which:
Figure 1 is a schematic representation of the head mountable eye monitor according to an embodiment of the present invention;
Figure 2 is a plan view of the head mountable eye monitor illustrated in figure 1, showing the path of the transmitted and reflected probe signal;
Figure 3 is a flowchart illustrating the steps associated with a method of monitoring pupillary response according to an embodiment of the present invention;
Figure 4 is a graphical representation of the variation of the signal intensity reflected from positions across a user's eye;
Figure 5 is a schematic representation of a helmet mountable eye monitor according to an embodiment of the present invention; and
Figure 6 is a schematic representation of a vehicle mountable eye monitor according to an embodiment of the present invention.

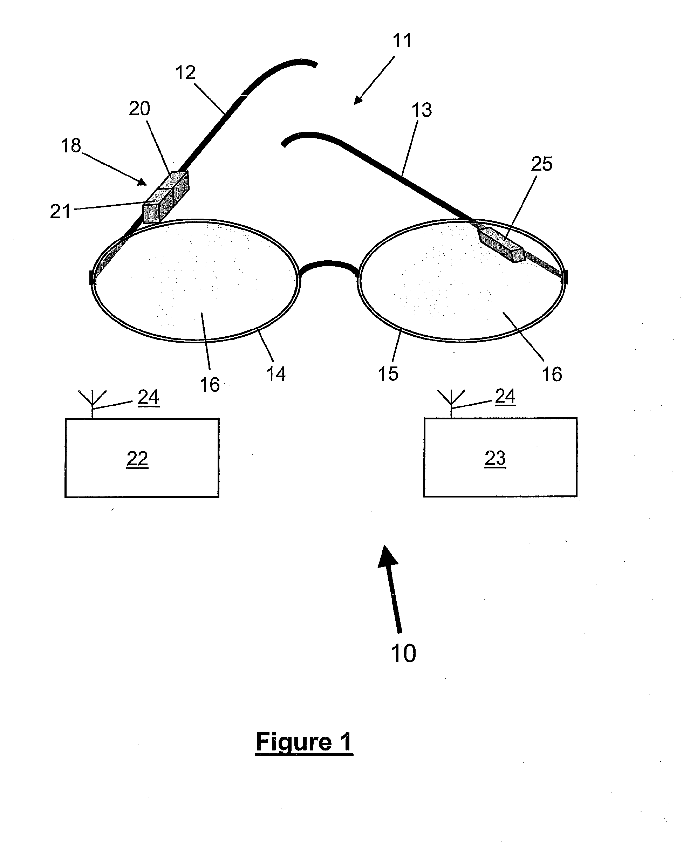

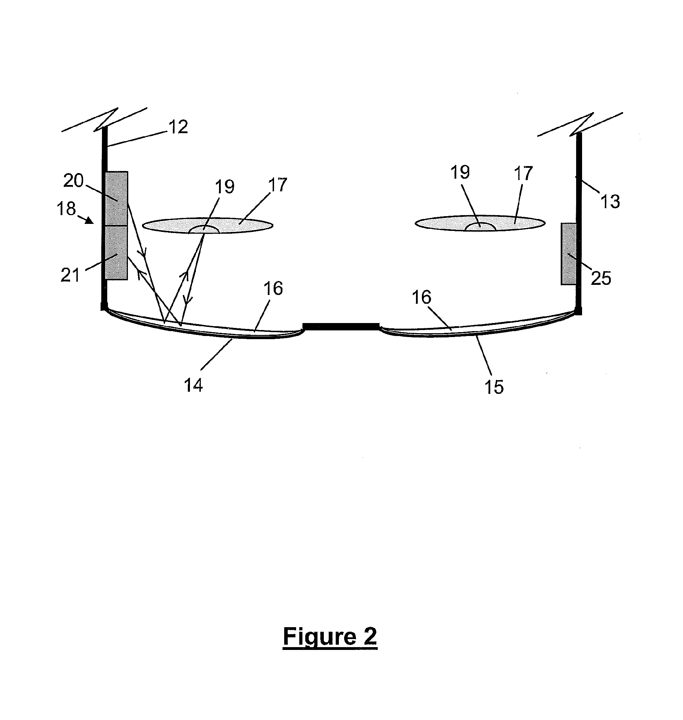

Referring to Figures 1 to 4, wherein like references have been used to indicate similar integers, there is illustrated a head mountable eye monitor 10 according to an embodiment of the present invention, which obviates the requirement for a subject being monitored, in this case a user (not shown), to cover both eyes in determining whether the user (not shown) has been exposed to harmful chemicals. The eye monitor 10 comprises a frame 11, such as a frame for a pair of spectacles, having a first and second arm 12, 13 which extend from a first and second support 14, 15 for an associated optical element 16, such as a lens. The arms 12, 13 are separately hingedly coupled at a proximal end thereof, to the respective optical element support 14, 15, which are separately arranged to support the associated optical element 16 which is arranged to be substantially in front of each of the user's eyes 17 when in use. It is envisaged that the optical elements 16 may be coated with a reflection coating (not shown) to prevent pilots (not shown) for example, from becoming blinded by laser pointers (not shown), for example.

The frame 11 is further arranged to support a tracking arrangement 18, which is arranged initially to locate a centre position of a pupil 19 of one or both of the user's eyes 17 and subsequently to autonomously track the movement of the user's eyes 17, and in particular movement of the pupils 19 of the user's eyes 17. The following description will refer to the tracking and monitoring the pupil 19 of one of the user's eyes 17, however, the skilled reader will recognise that the monitor of the present invention could also be used to track and monitor the pupil 19 of each of the user's eyes 17.

The tracking arrangement 18 comprises a transmitter 20, which is arranged to transmit an infrared probe signal substantially upon a user's eye 17 and a receiver 21, such as an infrared camera or sensor, which is arranged to receive an infrared signal which becomes reflected off the user's eye 17. The tracking arrangement 18 further comprises signal steering means (not shown) which is arranged to suitably direct the signal upon the user's eye 17 and to scan the probe signal across the user's eye 17.

The eye monitor 10 further comprises a processor 22 for processing signals received from the receiver 21 and a control unit 23 for controlling the operation of the transmitter 20 and receiver 21. The processor 22 and control unit 23 are communicatively coupled with the tracking arrangement 18 and may be supported upon the frame 11 or otherwise disposed within a cockpit (not shown) or upon a pilots helmet (not shown), for example. In the embodiment illustrated in figure 1, the processor 22 and control unit 23 are arranged in communication with the tracking arrangement 18 via a wireless communication link 24. The eye monitor 10 may further comprise a light sensor 25 mounted upon the frame 11 for example for monitoring the ambient light conditions, and is communicatively coupled with the processor 22 via the wireless communications link 24, for example.

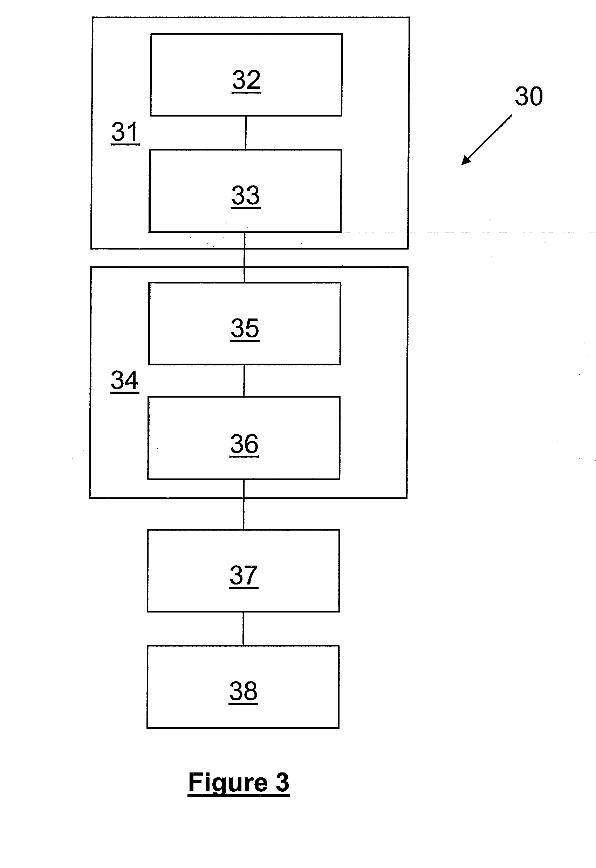

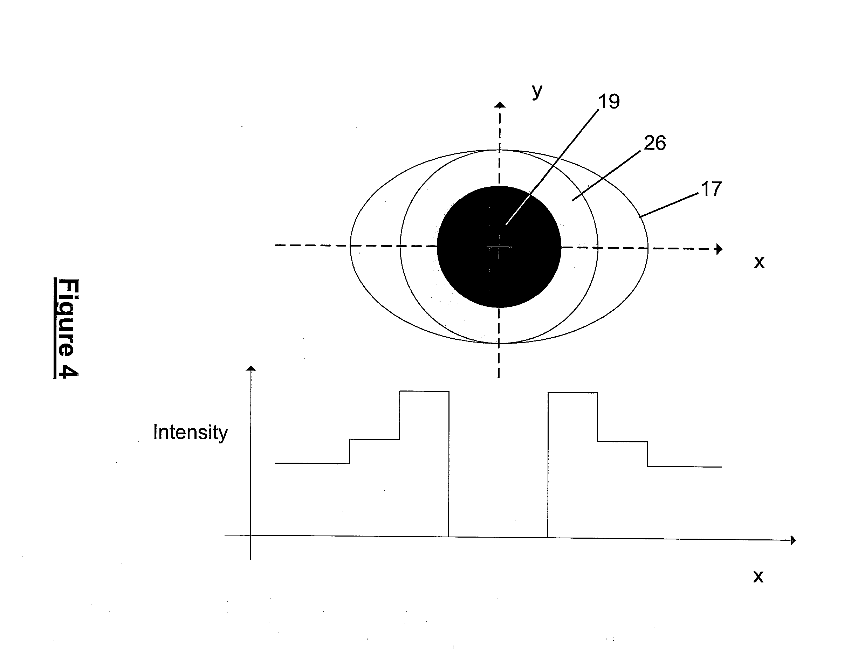

Referring to figure 3 of the drawings, there is illustrated a flowchart of the steps associated with a method 30 of monitoring pupillary response according to an embodiment of the present invention. In order to track and monitor the pupil of a user's eye 17, the tracking arrangement 18 is arranged to first locate the position of the centre of the user's pupil. This is performed during a pupil location step 31 in which the probe signal is scanned across the user's eye 17 at step 32 and the variation in reflected signal intensity in the x-direction and y-direction is monitored at step 33. The location of the centre position of the pupil 19 of the user's eye 17 is achieved by directing the infrared probe signal upon the optical element 16 disposed in front of the respective eye 17, such that the probe signal becomes reflected off the optical element 16 onto the respective pupil 19. The probe signal, which subsequently becomes reflected off the pupil 19 is further arranged to reflect off the optical element 16 onto the receiver 21. The receiver 21 subsequently communicates the received signal data via the wireless communications link 24 to the processor 22, which is arranged to determine the size of the pupil 19. The processor 22 further receives data from the light sensor 25 relating to the ambient lighting levels. The centre-point of the pupil position is determined by identifying the intersection of the x-axis and y-axe. A typical graphical representation of the variation in reflected light intensity is illustrated in figure 4 of the drawings.

Once the location of the centre position of the pupil 19 has been determined, the tracking arrangement 18 is subsequently arranged to track the movement of the user's pupil 19 at step 34. This is achieved by monitoring for changes in the intensity of the reflected signal, as the user moves their eye 17. As the tracking arrangement 18, tracks the movement of the user's eye 17, the probe signal is directed by the signal steering means (not shown) upon the user's eye 17 at step 35, and the processor 22 is arranged to process the signal reflected from the user's eye 17 to determine the size of the user's pupil 19 at step 36. This is performed by determining the range either side of the centre-point, which provides substantially the same reflected signal intensity, and may take place at a rate of 10-100 times per second. The signal reflected off the user's eye 17 is dependent upon the position on the eye 17 from which the signal becomes reflected from. The pupil 19 and iris 26 which surrounds the pupil, reflect the signal by different amounts. Accordingly, once the centre-point of the pupil has been identified, the pupil diameter can be measured by determining the range either side of the centre-point which provides substantially the same reflected signal intensity. The algorithm used by the processor 22 is arranged to provide a time average of the determined pupil size so that a reduction in error or false measurements can be achieved. Accordingly, by constantly monitoring the size of the user's pupil 19, a comprehensive record of the range of pupil sizes can be determined, which therefore allows for a rapid identification of unusual pupil activity.

Alternatively, the determined pupil size or time average pupil size may be compared with a calibrated range of acceptable pupil sizes. The calibrated range may be obtained by monitoring the range of pupil sizes for the user (not shown) under various controlled lighting conditions. As a further alternative, the processor 22 may be used to compare the determined pupil size with an average pupil diameter for a population group to which the user (not shown) belongs and for that particular lighting level.

If the measured pupil size is found to reside outside of an acceptable range at step 37, the head mountable eye monitor 10 is arranged to warn the user, such as the pilot (not shown), via an audible and/or visual alarm (not shown) at step 38. In this manner, the eye monitor 10 provides for a continuous and real time analysis of the user's eye (or eyes) 17 and in particular the pupil of the (or each) eye, to determine whether the user (not shown) has been exposed to harmful chemicals and without requiring a user (not shown) to break away from their normal duties or to cover their eyes 17.

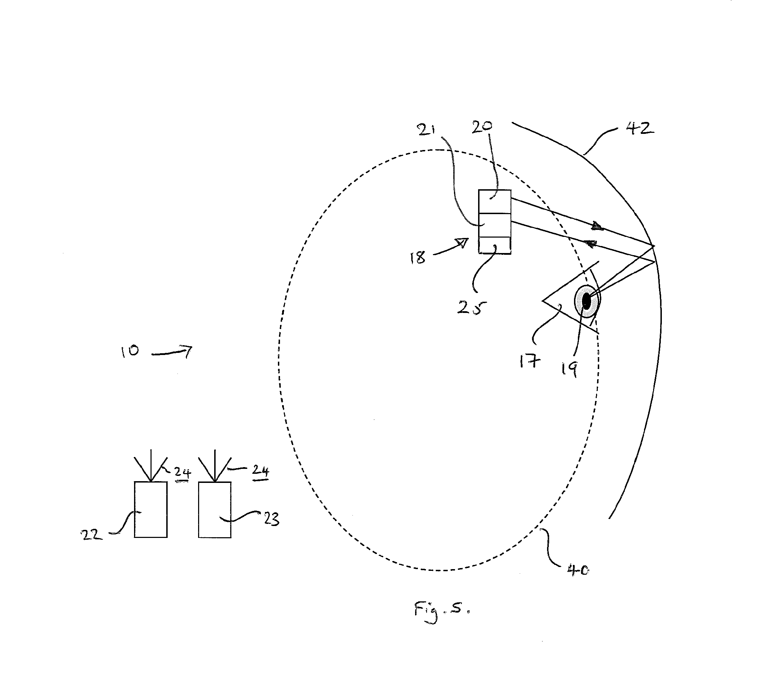

Referring to Figure 5, wherein like references have been used to indicate similar integers to those described with reference to Figures 1 to 4, there is illustrated a helmet mountable eye monitor 10 according to another embodiment of the present invention.

The eye monitor 10 comprises a tracking arrangement 18, which is arranged initially to locate a centre position of a pupil 19 of one or both of the eyes 17 of a wearer and subsequently to autonomously track the movement of the eyes 17 of the wearer, and in particular movement of the pupils 19 of the wearer's eyes 17. The following description will refer to the tracking and monitoring the pupil 19 of one of the wearer's eyes 17, however, the skilled reader will recognise that the monitor of the present invention could also be used to track and monitor the pupil 19 of each of the wearer's eyes 17. The tracking arrangement 18 is mounted at a suitable location on a helmet, not illustrated, to be donned by a wearer 40. The helmet includes a visor arrangement 42 arranged to be located in front of the eye or eyes 17 of the wearer 40. The visor arrangement 42 is arranged to allow the wearer, in this case a pilot of an aircraft, to view a forward scene through the visor arrangement. The visor arrangement 42 can also be arranged to present an image to overlay the forward scene viewed by the wearer 40. The image overlay can be conformal with the forward scene.

The tracking arrangement 18 comprises a transmitter 20, which is arranged to transmit an infrared probe signal substantially upon the wearer's eye 17 and a receiver 21, such as an infrared camera or sensor, which is arranged to receive an infrared signal which becomes reflected off the wearer's eye 17. The tracking arrangement 18 further comprises signal steering means (not shown) which is arranged to suitably direct the signal upon the wearer's eye 17 and to scan the probe signal across the wearer's eye 17.

The eye monitor 10 further comprises a processor 22 for processing signals received from the receiver 21 and a control unit 23 for controlling the operation of the transmitter 20 and receiver 21. The processor 22 and control unit 23 are communicatively coupled with the tracking arrangement 18 and may be disposed within a cockpit (not shown) or upon the helmet (not shown). In the embodiment illustrated in figure 5, the processor 22 and control unit 23 are arranged in communication with the tracking arrangement 18 via a wireless communication link 24. The eye monitor 10 may further comprise a light sensor 25 mounted within the helmet arranged to monitor the ambient light conditions, and is communicatively coupled with the processor 22 via the wireless communications link 24, for example.

The operation of the eye monitor 18 is substantially as that described with reference to figures 3 and 4, above.

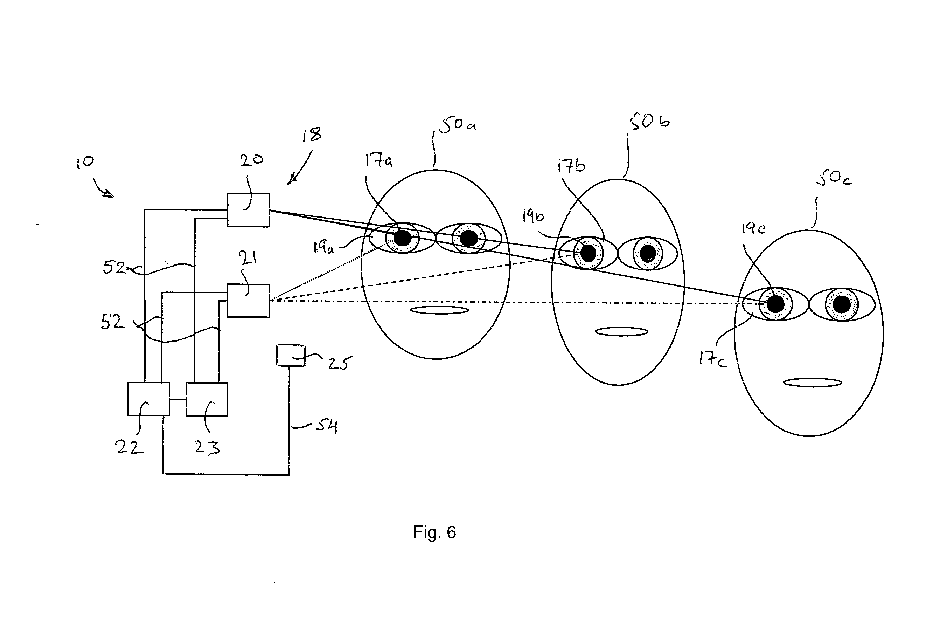

Referring to Figure 6, wherein like references have been used to indicate similar integers to those described with reference to Figures 1 to 4, there is illustrated an eye monitor 10 according to another embodiment of the present invention.

The eye monitor 10 comprises a tracking arrangement 18, which is arranged initially to locate a centre position of a pupil 19a, 19b or 19c of one or both of the eyes 17a, 17b or 17c of a plurality of subjects 50a to 50c to be monitored and subsequently to autonomously track the movement of the eyes 17a to 17c of the subjects 50a to 50c, and in particular movement of the pupils 19a to 19c of the eyes 17a to 17c of the monitored subjects 50a to 50c. The following description will refer to the tracking and monitoring the pupil 19 of one of the subjects 50a and associated eye 17a, however, the skilled reader will recognise that the monitor of the present invention could also be used to track and monitor the pupil 19a to 19c of each of the subjects 50a to 50c in turn. The tracking arrangement 18 is mounted at a suitable location to provide a field of view of the subjects 50a to 50c, for example the interior of a vehicle or public space, not illustrated.

The tracking arrangement 18 comprises a transmitter 20, which is arranged to transmit an infrared probe signal substantially upon the subject's 50a eye 17a and a receiver 21, such as an infrared camera or sensor, which is arranged to receive an infrared signal which becomes reflected off the subject's 50a eye 17. The tracking arrangement 18 further comprises signal steering means (not shown) which is arranged to suitably direct the signal upon the subject's eye 17a and to scan the probe signal across the subject's eye 17a.

The eye monitor 10 further comprises a processor 22 for processing signals received from the receiver 21 and a control unit 23 for controlling the operation of the transmitter 20 and receiver 21. The processor 22 and control unit 23 are communicatively coupled with the tracking arrangement 18 and may also be disposed within the interior of the vehicle (not shown) or public space (not shown). In the embodiment illustrated in figure 6, the processor 22 and control unit 23 are arranged in communication with the tracking arrangement 18 via communication links 52. The eye monitor 10 may further comprise a light sensor 25 mounted within the interior of the vehicle or public space which is arranged to monitor the ambient light conditions, and is communicatively coupled with the processor 22 via a communications link 54.

The operation of the eye monitor 18 is substantially as that described with reference to figures 3 and 4, above.

It will be understood by a person skilled in the art that the embodiments described herein are intended as examples only and that the separate integers of each embodiment may be combined with features of the other embodiments to provide an eye monitor, head or helmet mountable eye monitor or vehicle mountable eye monitor according to the invention as claimed.

## Claims

1. An eye monitor for monitoring the size of a pupil of an eye of a monitored subject, the monitor including tracking means for tracking movement of the pupil and means for determining the size of the pupil.

2. An eye monitor according to claim 1, wherein the tracking means includes a transmitter for transmitting a probe signal substantially upon the eye of the subject and a receiver for receiving a signal reflected off the eye of the subject.

3. An eye monitor according to claim 2, wherein the transmitter includes an infrared transmitter for transmitting a probe signal having a wavelength characteristic of infrared wavelengths.

4. An eye monitor according to claim 2 or 3, wherein the receiver includes an infrared sensor for receiving the probe signal reflected from the eye of the subject.

5. An eye monitor according to any of claims 2 to 4, wherein the tracking means further includes signal steering means for steering the transmitted signal upon the eye of the subject.

6. An eye monitor according to claim 5, wherein the signal steering means is further arranged to scan the probe signal across the eye of the subject.

7. An eye monitor according to claim 6, wherein the receiver is arranged to monitor the variation in intensity of the reflected signal.

8. An eye monitor according to claim 7, wherein the signal steering means is arranged to direct the probe signal onto the eye of the subject in accordance with the reflected signal intensity.

9. An eye monitor according to claim 7 or 8, wherein the means for determining the size of the users pupil including a processor which is arranged to process the monitored variation in intensity of the reflected signal.

10. An eye monitor according to any of claims 2 to 9, further including control means for controlling the operation of the transmitter and the receiver.

11. An eye monitor according to claim 10, wherein the control means and the processor are communicatively coupled with the transmitter and the receiver.

12. A head or helmet mountable eye monitor including an eye monitor according to any preceding claim, wherein the eye monitor is mounted upon a frame.

13. A head or helmet mountable eye monitor according to claim 12, wherein the frame is arranged to support an optical element in front of one or both of the eyes of the subject.

14. A head mountable eye monitor according to claim 13 as appended to claim 5, wherein the signal steering means is arranged to steer the probe signal upon the optical element, such that the probe signal will reflect off the optical element, substantially onto one or both of the eyes of the subject.

15. A vehicle mountable eye monitor including the eye monitor of claims 1 to 11, wherein the eye monitor is mounted to the structure of a vehicle.

16. A method of monitoring pupillary response, the method including:
tracking pupil movement to determine pupil position;
transmitting a probe signal substantially upon the pupil in response to the tracked position; and,
receiving a signal reflected off the pupil.

17. A method according to claim 16, further including processing the received signal to determine the pupil size.

18. A method according to claim 17, further including comparing the measured pupil size with a calibrated acceptable pupil range.
